# EUROPÄISCHE PATENTANMELDUNG

(11) **EP 0 945 104 A1**
(43) Veröffentlichungstag der Anmeldung: **29.09.1999**
(21) Anmeldenummer: 98810261.2
(22) Anmeldetag: 25.03.1998
(51) Int. Cl.: A61B 8/12

(54) **System und Verfahren zur Visualisierung von Aktivitäten eines Organs**

(71) Anmelder: Sulzer Osypka GmbH, 79639 Grenzach-Wyhlen (DE)
(72) Erfinder: Geistert, Wolfgang, Dr., 79618 Rheinfelden-Herten (DE)
(74) Vertreter: Sulzer Management AG

(57) **Zusammenfassung**

Ein System zur Visualisierung von Aktivitäten eines Organs (10) umfasst eine bildgebende Ultraschallvorrichtung (2) zum Bereitstellen von ersten Signalen, eine Rekonstruktionseinheit (7), welche aus den ersten Signalen eine bildliche Darstellung des Organs (10) erstellt, eine Messvorrichtung (3) zum örtlich aufgelösten Erfassen zweiter Signale, welche zweite Signale repräsentativ für die Aktivität des Organs (10) sind sowie eine Zuordnungseinheit (6), welche den zweiten Signalen jeweils den örtlichen Bereich des Organs (10) zuordnet, für dessen Aktivität das jeweilige zweite Signal repräsentativ ist sowie eine Visualisierungseinheit (8), welche aus der bildlichen Darstellung, den zweiten Signalen sowie der jeweils zugehörigen Ortsinformation ein Bild des Organs (10) erzeugt, wobei das Bild sowohl die bildliche Darstellung als auch die Aktivität des Organs (10) in einander örtlich zugeordneter Weise umfasst.

## Beschreibung

Die Erfindung betrifft ein System sowie ein verfahren zur Visualisierung von Aktivitäten eines Organs. Insbesondere betrifft die Erfindung ein System und ein Verfahren zur Visualisierung der Aktivität des Herzens.

Bei der Diagnose und der Behandlung von Herzrhythmusstörungen werden heute in der Kardiologie häufig Katheter eingesetzt, die in das Herz eingeführt werden, um dort zum einen elektrophysiologische Signale zu erfassen und zum anderen gezielt Ablationen kardialen Gewebes durchzuführen, welches die Ursache der Herzrhythmusstörungen ist. Dazu ist es bekannt, mit einem Katheter an verschiedenen Positionen im Herzen mittels einer Elektrode die elektrische Aktivität des Herzens lokal zu erfassen. Der Katheter kann weiterhin spezielle Sensoren enthalten, die es erlauben, seine jeweilige Lage im Herzen zu detektieren, beispielsweise mittels spezieller elektromagnetischer Felder. Anhand der so erfassten Daten lässt sich nach und nach eine Art dreidimensionaler Darstellung der elektrischen Aktivitäten des Herzens aufbauen, die beispielsweise von einem Arzt analysiert werden kann.

Derart arbeitende Vorrichtungen haben Jedoch den Nachteil, dass die örtlichen Informationen relativ ungenau und lückenhaft sind, weil sie nur von solchen Positionen vorliegen, an denen sich der Katheter bereits einmal befunden hat. Weiterhin bringt die Reproduzierbarkeit einer bestimmten Katheterposition Schwierigkeiten mit sich, weil sich beispielsweise die örtlichen Gegebenheiten aufgrund des Herzschlags ändern. Zudem kann es vorkommen, dass an wichtigen Orten des Herzens keine Signale aufgenommen wurden, wodurch das räumlich aufgelöste Elektrokardiogramm (EKG) erhebliche Fehler aufweisen kann.

Es ist daher eine Aufgabe der Erfindung, ein System und ein Verfahren zur Visualisierung von Aktivitäten eines Organs, insbesondere des Herzens, bereitzustellen, die eine genaue und zuverlässige Visualisierung der Organaktivität ermöglichen. Insbesondere sollen es das System und das Verfahren ermöglichen, den räumlichen und zeitlichen Verlauf der Organaktivitäten in übersichtlicher Weise darzustellen.

Die diese Aufgaben lösenden Gegenstände der Erfindung sind durch die Merkmale der unabhängigen Ansprüche der jeweiligen Kategorie gekennzeichnet.

Erfindungsgemäss wird also vorgeschlagen, mittels einer bildgebenden Ultraschallvorrichtung sowie mit einer Rekonstruktionseinheit eine bildliche Darstellung des Organs oder von Teilen des Organs zu erstellen, sowie mit einer Messvorrichtung zweite Signale zu erfassen, die repräsentativ für die Aktivität des Organs sind. Diese Messvorrichtung umfasst z. B. in einer bevorzugten Ausführungsform einen Katheter, mit dem die lokale Aktivität des Organs, beispielsweise in Form lokaler elektrokardiografischer (EKG-) Daten, die als zweite Signale dienen, an mehreren Positionen des Organs erfasst wird.

Erfindungsgemäss ordnet eine Zuordnungseinheit den zweiten Signalen jeweils als Ortsinformation den örtlichen Bereich des Organs zu, für dessen Aktivität das jeweilige zweite Signal repräsentativ ist. Schliesslich werden in einer Visualisierungseinheit die bildliche Darstellung, die zweiten Signalen sowie die jeweils zugehörigen Ortsinformationen zu einem Bild kombiniert, das sowohl die bildliche Darstellung als auch die Aktivität des Organs in einander örtlich zugeordneter Weise umfasst.

Die Ultraschallvorrichtung dient folglich dazu, eine bildliche bzw. geometrische Darstellung des Organs zu erzeugen, die mit den lokal aufgelösten zweiten Signalen, also den Aktivitätssignalen, derart kombiniert oder überlagert wird, dass sich ein Bild ergibt, welches die geometrische bildliche Darstellung des Organs zeigt und zusätzlich die lokale Aktivität des Organs als Funktion des Orts.

Da die geometrische bildliche Darstellung des Organs primär nicht auf Ortsinformationen beruht, die von der Messvorrichtung für die zweiten Signale kommen, sondern im wesentlichen unabhängig von dieser Messvorrichtung mittels einer bildgebenden Ultraschallvorrichtung erstellt wird, ermöglicht das erfindungsgemässe System bzw. Verfahren eine deutlich genauere, örtlich höher aufgelöste und vollständigere bildliche Darstellung des Organs, in welche dann in der Folge die Aktivitätssignale eingearbeitet werden können. Hierdurch lässt sich eine sehr übersichtliche, zuverlässige und örtlich aufgelöste Visualisierung der Organaktivität realisieren.

Bei dem erfindungsgemässen Verfahren zur Visualisierung von Aktivitäten eines Organs, insbesondere des Herzens, wird mittels Ultraschallmesssignalen eine bildliche Darstellung des Organs oder von Teilen des Organs, insbesondere eine dreidimensionale bildliche Darstellung, erzeugt. Ferner werden zweite Signale, insbesondere elektrischer Signale, welche repräsentativ für die Aktivität des Organs sind, mit der genannten bildlichen Darstellung zu einem Bild des Organs oder von Teilen des Organs kombiniert, wobei das Bild sowohl die bildliche Darstellung als auch die Aktivität des Organs in einander örtlich zugeordneter Weise umfasst.

Vorteilhafterweise wird das Bild kontinuierlich oder in diskreten Zeitabständen aktualisiert, indem aktuelle zweite Signale mit der bildlichen Darstellung neu kombiniert werden. Durch diese Massnahme ist neben der räumlichen Information bezüglich der Aktivität auch der zeitliche Verlauf der Aktivität als Funktion des Orts darstellbar.

Weitere vorteilhafte Massnahmen und bevorzugte Ausgestaltungen der Erfindungsgegenstände ergeben sich aus den abhängigen Ansprüchen.

Im folgenden wird die Erfindung sowohl in Bezug auf die verfahrenstechnischen als auch in Bezug auf die apparativen Aspekte anhand der Zeichnung und anhand von Ausführungsbeispielen näher erläutert. In der schematischen nicht massstäblichen Zeichnung zeigen:
- Fig. 1:: eine symbolische Darstellung eines Ausführungsbeispiels des erfindungsgemässen Systems, und
- Fig. 2:: eine schematische Schnittdarstellung eines Katheters einer Messvorrichtung des erfindungsgemässen Systems.

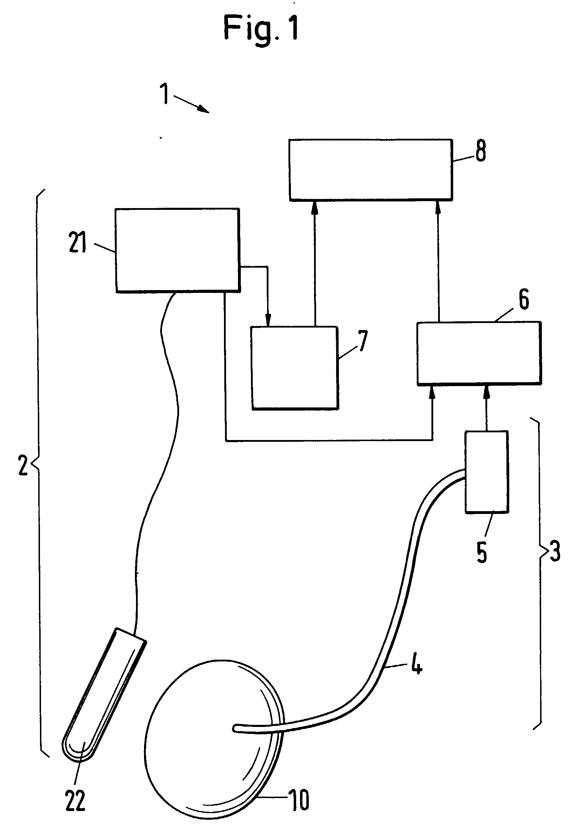

Fig. 1 zeigt in einer symbolischen Darstellung die wesentlichen Komponenten eines Ausführungsbeispiels des erfindungsgemässen Systems, das gesamthaft mit dem Bezugszeichen 1 bezeichnet ist. Das erfindungsgemässe System 1 zur Visualisierung von Aktivitäten eines Organs 10 umfasst eine bildgebenden Ultraschallvorrichtung 2 zum Bereitstellen von ersten Signalen, eine Rekonstruktionseinheit 7, welche aus den ersten Signalen eine bildliche Darstellung des Organs 10 oder von Teilen des Organs 10 erstellt, eine Messvorrichtung 3 zum örtlich aufgelösten Erfassen zweiter Signale, welche repräsentativ für die Aktivität des Organs 10 sind, sowie eine Zuordnungseinheit 6, welche den zweiten Signalen jeweils als Ortsinformation den örtlichen Bereich des Organs 10 zuordnet, für dessen Aktivität das jeweilige zweite Signal repräsentativ ist. Das System 1 umfasst ferner eine Visualisierungseinheit 8, welche aus der bildlichen Darstellung, den zweiten Signalen sowie der jeweils zugehörigen Ortsinformation ein Bild des Organs 10 oder von Teilen des Organs 10 erzeugt, wobei das Bild sowohl die bildliche Darstellung als auch die Aktivität des Organs 10 in einander örtlich zugeordneter Weise umfasst.

Im folgenden wird mit beispielshaftem Charakter auf den für die Praxis wichtigen Fall Bezug genommen, dass das Organ 10, dessen Aktivität visualisiert werden soll, das Herz ist, und dass die zweiten Signale, welche repräsentativ für die Aktivität des Organs sind, elektrische Aktivitätssignale (EKG-Signale) des Herzens 10 sind, welche mittels eines Katheters 4 intrakardial erfasst werden.

Die bildgebende Ultraschallvorrichtung 2 umfasst einen Messkopf 22 zum Aussenden von Ultraschallsignalen und zum Empfangen der von der abzubildenden Körperstruktur reflektierten Echosignale. Der Messkopf 22 wird von einem Ultraschallmodul 21 angesteuert. Der Messkopf 22 übermittelt die empfangenen Echosignale an das Ultraschallmodul 21, welches diese Echosignale vorverarbeitet, z. B. verstärkt und/oder filtert, und sie anschliessend als erste Signale an die Rekonstruktionseinheit 7 weiterleitet. Die Rekonstruktionseinheit 7 wandelt die ersten Signale mittels an sich bekannter Prozeduren zur zwei- bzw. dreidimensionalen Bildgebung in ein Format um, welches eine bildliche Darstellung des Organs 10 ermöglicht.

Vorzugsweise sind die Ultraschallvorrichtung 2 und die Rekonstruktionseinheit 7 derart ausgestaltet, dass sie eine dreidimensionale (3D-) bildliche Darstellung des Organs 10 oder von Teilen des Organs ermöglichen. Mit "dreidimensionaler" Darstellung ist dabei gemeint, dass die Darstellung tatsächlich dreidimensional ist oder, dass sie zwar eben ist aber einen dreidimensionalen Eindruck vermittelt, z. B. mittels Methoden der räumlichen oder perspektivischen Darstellung oder der Projektion.

In der europäischen Patentanmeldung Nr. 97811021.1 wird eine Ultraschallvorrichtung offenbart, die sich dadurch auszeichnet, dass sie in extrem kurzer Zeit - nämlich näherungsweise in Echtzeit - dreidimensionale bildliche Darstellungen des Herzens ermöglicht. Die dreidimensionale Abbildung des Herzens mittels Ultraschall erfolgt dabei vorzugsweise transösophagal. Aufgrund der dadurch geschaffenen Möglichkeit, das Herz nahezu kontinuierlich mittels stets aktueller dreidimensionaler Darstellungen abzubilden, ist ein solches Ultraschallsystem, auch im Hinblick auf die Navigation und die Lokalisierung von Instrumenten im Herzen, für das erfindungsgemässe System bzw. Verfahren besonders gut geeignet. Bezüglich der detaillierten Beschreibung der Ausgestaltung einer solchen schnellen bildgebenden 3D-Ultraschallvorrichtung wird auf die europäische Patentanmeldung Nr. 97811021.1 verwiesen.

Es versteht sich, dass ausser diesen bevorzugten Ultraschallvorrichtungen auch alle anderen an sich bekannten bildgebenden Ultraschallgeräte für das erfindungsgemässe System bzw. Verfahren eingesetzt werden können. Dabei ist es nicht notwendig, dass das Ultraschallgerät für die dreidimensionale Bildgebung ausgelegt sein muss. Das erfindungsgemässe System bzw. Verfahren lässt sich durchaus auch mit solchen Ultraschallgeräten betreiben, die für zweidimensionale Ultraschalldarstellungen ausgestaltet sind.

Die Zuordnungseinheit 6 kann auch in die Ultraschallvorrichtung 2 integriert sein. Ferner können die Rekonstruktionseinheit 7 und/oder das Ultraschallmodul 21 und/oder die Visualisierungseinheit 8 und/oder die Zuordnungseinheit 6 eine bauliche Einheit bilden, die Teil der Ultraschallvorrichtung 2 ist.

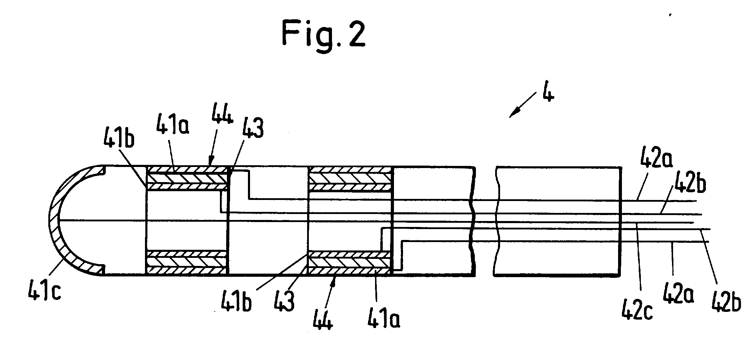

Die Messvorrichtung 3 zum Erfassen der zweiten Signale, hier beispielsweise der elektrischen Aktivitätssignale des Herzens 10, umfasst in dem Ausführungsbeispiel den Katheter 4, der zur lokalen Erfassung der Aktivitätssignale in das Herz eingeführt wird, sowie eine Ansteuer- und Messeinheit 5, welche die von dem Katheter kommenden Signale empfängt. Der Katheter 4 ist mit Hilfe an sich bekannter Mittel im Innern des Herzens navigierbar. Zum besseren Verständnis zeigt Fig. 2 eine mögliche Ausgestaltung des Katheters 4 in einer schematischen Schnittdarstellung. Er umfasst mindestens eine Elektrode 41a, 41c zur lokalen Erfassung der elektrischen Aktivitätssignale des Herzens 10. In Fig. 2 sind mit beispielhaftem Charakter drei Elektroden 41a,41c zur Erfassung der Aktivitätssignale dargestellt, wobei zwei ringförmige Elektroden 41a an der Mantelfläche des Katheters 4 angeordnet sind und eine Elektrode 41c am distalen Ende des Katheters 4. Die Elektroden 41a, 41c können als Kontakt-Elektroden oder für die kontaktlose Erfassung der Aktivitätssignale ausgestaltet sein. Die Elektroden 41a, 41c sind über Verbindungsleitungen 42a,42c mit der Ansteuer- und Messeineinheit 5 verbunden, um die von ihnen erfassten Aktivitätssignale an die Ansteuer- und Auswerteeinheit 5 zu übermitteln. Die Erfassung der Aktivitätssignale mittels der Elektroden 41a, 41c kann sowohl unipolar als auch bipolar erfolgen.

Vorzugsweise umfasst der Katheter 4 auch mindestens einen Ultraschallwandler 44 zum Empfangen und/oder Aussenden von Ultraschallsignalen, weil dadurch eine einfache und sichere Lokalisierung des Katheters 4 bzw. der Elektroden 41a, 41c im Herzen 10 mittels Ultraschall ermöglicht wird. Diese Lokalisierung wird weiter hinten noch detailliert beschrieben. Jeder Ultraschallwandler 44 umfasst eine Wandlerschicht 43 aus piezoelektrischem Material, die zwischen den beiden Elektroden 41a und 41b eingebettet ist. Bei der in Fig. 2 dargestellten Ausführungsform sind die Ultraschallwandler 44 jeweils ringförmig ausgestaltet und so angeordnet, dass sie den Katheter 4 entlang seines Umfangs umfassen. Die Elektoden 41a und 41b sind in Fig. 2 übertrieben dick dargestellt; üblicherweise sind die Elektroden 41a und 41b leitfähige Beschichtungen auf den Oberflächen der Wandlerschichten 43. Mittels der Elektoden 41a, 41b können die von der Wandlerschicht 43 erzeugten elektrischen Signale abgegriffen und über die Verbindungsleitungen 42a,42b der Ansteuer- und Messeinheit 5 zugeführt werden. In umgekehrter Weise können die Wandlerschichten 43 mittels elektrischer Impulse, die von der Ansteuer- und Messeinheit 5 über die Verbindungsleitungen 42a, 42b und die Elektroden 41a,41b auf die Wandlerschichten 43 übertragen werden, zum Aussenden von Ultraschallsignalen angeregt werden.

Wie dies die voranstehende Beschreibung schon andeutet, kommt den Elektroden 41a eine doppelte Funktion zu. Sie dienen zum einen der lokalen Erfassung der Aktivitätssignale des Herzens 10 und bilden zum anderen einen Teil des Ultraschallwandlers 44. Die Massnahme, dass der Ultraschallwandler 44 auch als Elektrode zum lokalen Erfassen der elektrischen Aktivität dient, ist vorteilhaft, weil sie eine besonders platzsparende und kostengünstige Ausgestaltung des Katheters 4 ermöglicht.

Ausser der ringförmigen Ausgestaltung des Ultraschallwandlers 44 ist natürlich auch z. B. eine plattenförmige Ausgestaltung möglich, das heisst der Ultraschallwandler 44 umfasst nicht den gesamten Umfang des Katheters 4. Ferner ist es möglich, auf einem Katheter 4 sowohl plattenförmige als auch ringförmige Ultraschallwandler 44 vorzusehen. Natürlich kann der Ultraschallwandler 44 auch andere Formen aufweisen und/oder im Innern des Katheters angeordnet sein.

Es versteht sich, dass der Ultraschallwandler 44 und die Elektroden 41a, die zum Erfassen der Aktivität dienen, auch baulich voneinander getrennt sein können.

Im Hinblick auf eine möglichst effiziente und rasche Erfassung der lokalen Aktivitätssignale ist es vorteilhaft, wenn der Katheter 4, wie in Fig. 2 gezeigt, hierfür mehrere Elektroden 41a,41c umfasst, sodass mehrere Aktivitätssignale von unterschiedlichen lokalen Bereichen des Herzens 10 gleichzeitig erfassbar sind. Die Elektroden 41a, 41c können dabei eindimensional, d.h. entlang einer Linie, aber insbesondere auch zweidimensional, d. h. auf einer Fläche, oder dreidimensional, beispielsweise auf einem solchen Katheter, dessen distaler Endbereich räumlich aufspannbar ist, angeordnet sein. Auch ist es möglich, die leitfähige, als Elektrode 41a des Ultraschallwandlers 44 dienende Beschichtung der Wandlerschicht 43 in mehrere elektrisch gegeneinander isolierte Teilbereiche aufzuteilen, sodass durch eine solche leitfähige Beschichtung mehrere Elektroden zur Erfassung der AXtivitätssignale realisiert werden.

Insbesondere bei mehreren Elektroden 41a,41c ist es vorteilhaft, wenn auch mehrere Ultraschallwandler 44 vorgesehen sind, um die Lage des Katheters im Raum, und damit die Lage der einzelnen Elektroden 41a, 41c, einfacher und genauer feststellen zu können. Alternativ oder ergänzend ist es möglich, einen Ultraschallwandler 44 so anzuordnen, dass er bezüglich des Katheters 4 beweglich ist. Dadurch ist es möglich, den Ultraschallwandler 44 an verschiedene Positionen bezüglich des Katheters 4 zu bewegen, um somit jeweils die räumliche Lage der einzelnen Elektroden zu ermitteln.

Zum Betreiben des Systems wird zunächst der Messkopf 22 der Ultraschallvorrichtung 2 in der Nähe der abzubildenden Körperstruktur, hier also des Herzens 10, plaziert. Vorzugsweise wird der Messkopf 22 dazu in den Ösophagus eingeführt, weil sich von dort aus eine besonders gute bildliche Darstellung des Herzens erzielen lässt. Mittels der vom Messkopf 22 empfangenen Ultraschallechosignale erstellt das Ultraschallmodul 21 in Verbindung mit der Rekonstruktionseinheit 7 eine vorzugsweise dreidimensionale bildliche Darstellung des Herzens. Bei der Verwendung solcher schnellen bildgebenden Ultraschallvorrichtungen 2 wie sie in der bereits zitierten europäischen Patentanmeldung 97811021.1 offenbart sind, kann diese bildliche Darstellung näherungsweise in Echtzeit erfolgen, wodurch kontinuierlich eine aktuelle Visualisierung des Herzens ermöglicht wird.

Der Katheter 4 wird in an sich bekannter Weise in das Herz 10 eingeführt. Je nachdem, wie viele Elektroden 41a,41c der Katheter 4 zur Erfassung der elektrischen Aktivitätssignale aufweist, wird er entweder an verschiedene Stellen im Herzen 10 navigiert, um dort sukzessive jeweils die lokale Aktivität, z. B. auch kontaktlos, zu erfassen, oder es werden gleichzeitig an mehreren Positionen des Herzens 10 die lokalen Aktivitäten erfasst. Die Aktivitätssignale werden über die Verbindungsleitungen 42a, 42b an die Ansteuer- und Messeinheit 5 weitergeleitet.

Bei einer dreidimensionalen bildlichen Darstellung des Herzens 10 in Echtzeit lässt sich die aktuelle Lage und/oder Position des Katheters 4 im Herzen 10 prinzipiell auch ohne zusätzliche Hilfsmittel in der 3D-Darstellung erkennen. Somit kann beispielsweise das Bedienpersonal den erfassten Aktivitätssignalen jeweils die Ortsinformation, das heisst den örtlichen Bereich des Herzens 10, für den das jeweils erfasste Aktivitätssignal repräsentativ ist, manuell zuordnen, indem die aktuelle Lage des Katheters 4 in die Zuordnungseinheit 6 eingegeben wird.

Vorzugsweise erfolgt die Lokalisierung des Katheters 4 im Herzen 10 aber mittels Ultraschall. Hierfür umfasst der Katheter 4 - wie bereits erwähnt - mindestens einen Ultraschallwandler 44. Bei der folgenden Beschreibung wird der Einfachheit halber und, weil es zum Verständnis ausreicht, angenommen, dass nur ein Ultraschallwandler 44 am Katheter 4 vorgesehen ist. Natürlich gelten diese Erläuterungen in sinngemäss gleicher Weise auch für mehrere Ultraschallwandler 44 auf dem Katheter 4.

Der Ultraschallwandler 44 kann entweder für den passiven oder für den aktiven Betrieb ausgestaltet sein. "Passiv" bedeutet, dass der Ultraschallwandler 44 nur für den Empfang von Ultraschallsignalen verwendet wird, "aktiv" bedeutet, dass er selbst Ultraschallsignale aussenden kann.

Zum Erzeugen der dreidimensionalen bildlichen Darstellung sendet der Messkopf 22 der Ultraschallvorrichtung 2 Ultraschallsignale aus, die unter anderem auch den Ultraschallwandler 44 des Katheters treffen können. Beim passiven Betrieb des Ultraschallwandlers 44 sendet dieser, wenn er von einer Ultraschallwelle getroffen wird, eine entsprechende Information an die Zuordnungseinheit 6 oder das Ultraschallmodul 21. Da bekannt ist, zu welchen Zeitpunkten und in welche Richtung von dem Messkopf 22 Ultraschallwellen oder - impulse ausgesendet wurden, wie gross die Ausbreitungsgeschwindigkeit des Ultraschalls ist und wann ein Ultraschallechosignal vom Messkopf 22 empfangen wurde, kann der Zeitpunkt des Auftreffens einer Ultraschallwelle auf den Ultraschallwandler 44 im Katheter 4 einer räumlichen Lage des Ultraschallwandlers 44 und damit der Elektroden 41a, 41c zugeordnet werden. Somit kann auch dem Aktivitätssignal die Ortsinformation zugeordnet werden, das heisst, derjenige örtliche Bereich des Herzens 10, für dessen Aktivität das Signal repräsentativ ist.

Wird der Ultraschallwandler 44 aktiv betrieben, so wandelt er einen empfangenen Ultraschallimpuls, der vom Messkopf 22 kommt, in ein elektrisches Signal um und übermittelt dieses an einen Transponder, der beispielsweise in der Ansteuer- und Messeinheit 5 integriert sein kann oder als eine eigene bauliche Einheit realisiert ist. Der Transponder erzeugt dann ein Sendesignal und schickt dieses an den Ultraschallwandler 44, der daraufhin ein Ultraschallsignal aussendet. Dieses Ultraschallsignal wird vom Messkopf 22 empfangen und an das Ultraschallmodul 21 weitergeleitet. Das vom Ultraschallwandler 44 ausgesendete Ultraschallsignal weist bevorzugt eine besondere Charakteristik auf, wie beispielsweise eine Modulation, eine besondere Dauer oder Intensität, damit es besonders einfach von den Echosignalen, die von der abzubildenden Körperstruktur stammen, unterscheidbar ist. Somit kann die Ultraschallvorrichtung 2 die räumliche Lage des Ultraschallwandlers 44 und damit auch die Lage der Elektroden 41a, 41c feststellen. Auch auf diese Weise kann jedem Aktivitätssignal seine spezifische Ortsinformation zugeordnet werden.

Mittels des Ultraschallwandlers 44 kann somit sowohl im passiven als auch im aktiven Betrieb eine automatische Ermittlung der Lage des Katheters 4 im Herzen 10 erfolgen. Denn die Zuordnungseinheit 6 kann, gegebenenfalls in Verbindung mit der Ultraschallvorrichtung 2, mit Hilfe der Signale, die von dem oder den Ultraschallwandler(n) 44 kommen, jedem zweiten Signal, hier also jedem lokalen Aktivitätssignal, automatisch die zugehörige Ortsinformation zuordnen, die angibt, für welchen lokalen Bereich des Herzens 10 das jeweilige Aktivitätssignal repräsentativ ist.

Insbesondere bei mehreren Elektroden 41a, 41c ist es vorteilhaft, auch mehrere Ultraschallwandler 44 am oder im Katheter 4 vorzusehen, um dessen räumliche Lage und Orientierung im Herzen 10 zu detektieren. Dadurch wird nämlich eine besonders einfache und sichere automatische Verbindung zwischen der Geometrie des Herzens 10 und den elektrischen Aktivitätssignalen ermöglicht.

Nachdem die Zuordnungseinheit 6 jedem Aktivitätssignal seine Ortsinformation zugeordnet hat, werden die Aktivitätssignale zusammen mit ihrer Ortsinformation an die Visualisierungseinheit 8 übermittelt. Diese erzeugt dann aus der geometrischen Form, das heisst, den von der Rekonstruktionseinheit 7 gelieferten Daten für die bildliche Darstellung, und aus den Aktivitätssignalen mit zugeordneter Ortsinformation ein Bild des Herzens 10, wobei das Bild sowohl die bildliche Darstellung als auch die Aktivitätssignale in einander örtlich zugeordneter Weise umfasst. Dies bedeutet, dass die elektrischen Aktivitäten des Herzens der dreidimensionalen bildlichen Darstellung überlagert werden. Bei einer bevorzugten Variante ist zusätzlich auch die örtliche Lage des Katheters 4 im Herzen 10 in dem Bild enthalten.

Die Darstellung des Bilds kann auf verschiedene Weisen erfolgen. Beispielsweise kann die zeitliche Ausbreitung des EKGs in Zahlen oder Symbolen an dem jeweils zugehörigen Ort dargestellt werden. Auch ist es möglich, das zeitlich und räumlich aufgelöste EKG mittels Falschfarben darzustellen, indem jeder Verzögerungszeit relativ zu einem vorgebbaren Anfangszeitpunkt eine bestimmte Farbe zugeordnet wird. Ferner ist es möglich, die Ausbreitung der Aktivität als sich bewegende Wellenfront darzustellen.

Das Bild mit der vorzugsweise dreidimensionalen Darstellung und den Aktivitätssignalen kann mit Hilfe verschiedener Visualisierungmedien wie zum Beispiel einem Monitor, einem sogenannten Head Mounted Display (HMD), oder einem Bildschirm - gegebenenfalls in Verbindung mit einer 3D-Brille - erfolgen.

Bezüglich der Aktualisierung des Bilds, entweder kontinuierlich oder in diskreten Zeitabständen, sind mehrere Varianten möglich. Beispielsweise kann die vorzugsweise dreidimensionale bildliche Darstellung nur einmal erstellt werden, und die jeweils aktuellen zweiten Signale werden immer wieder neu mit dieser bildlichen Darstellung kombiniert bzw. in diese eingearbeitet. Auch ist es möglich die bildliche Darstellung auf Anforderung oder in bestimmbaren diskreten Zeitabständen zu erneuern. Ferner besteht die Möglichkeit, insbesondere bei solchen schnellen Ultraschallsystemen 2, wie sie in der bereits zitierten europäischen Patentanmeldung Nr. 97811021.1 offenbart sind, die vorzugsweise dreidimensionale bildliche Darstellung näherungsweise in Echtzeit, also kontinuierlich, zu aktualisieren. Weiterhin ist es auch möglich, nur Teile der bildlichen Darstellung kontinuierlich oder in diskreten Zeitabständen zu aktualisieren. Beispielsweise können solche Teile der bildlichen Darstellung aktualisiert werden, die Bereiche des Herzens wiedergeben, die von besonderem Interesse sind, z. B. der Bereich, in dem sich momentan der Katheter befindet.

Auch wenn das erfindungsgemässe System vorzugsweise für die transösophagale Herzabbildung und die intrakardiale EKG-Messung ausgestaltet ist, so sind durchaus auch andere Ausführungsformen möglich. Beispielsweise kann die Erfassung der Ultraschallsignale für die bildliche Darstellung auch von ausserhalb des Körpers erfolgen, also ohne den Messkopf 22 in den Körper einzuführen. Ferner kann die Messvorrichtung 3 auch so ausgestaltet sein, dass sie die zweiten Signale (Aktivitätssignale) von ausserhalb des Körpers aus erfassen kann, beispielsweise in Form eines Oberflächen-EKGs. Die Messvorrichtung 3 umfasst dann beispielsweise mindestens eine Elektrode zum lokalen Erfassen der elektrischen Aktivität des Herzens oder Organs 10, wobei diese Elektrode auf der Körperoberfläche angebracht wird.

Neben der elektrophysiologischen Untersuchung des Herzens 10 ist das erfindungsgemässe System bzw. Verfahren auch vorteilhaft für Ablationsbehandlungen einsetzbar. Als Energieform zur Denaturierung des Gewebes wird häufig Hochfrequenz-Energie eingesetzt. Bei der vorne beschriebenen Ausgestaltung des erfindungsgemässen Systems können für solche Behandlungen die Elektroden 41a, 41c des Katheters 4 oder eine dieser Elektroden 41a, 41c auch als Hochfrequenz-Energiequelle für die Ablation ausgestaltet sein. Somit ist neben der Diagnose auch die Behandlung insbesondere von Herzrhythmusstörungen, möglich. Es versteht sich, dass bei derartigen Ausgestaltungen entsprechende Steuer- und Versorgungseinrichtungen für die Hochfrequenz vorgesehen sind. Insbesondere auch bei Ablationsbehandlungen ist die schnelle Ultraschallvorrichtung, die eine dreidimensionale Bildgebung des Herzens einschliesslich des Katheters näherungsweise in Echtzeit ermöglicht, besonders vorteilhaft, weil hierdurch eine sichere und einfache Lokalisierung und Navigierung des Katheters 4 im Herz 10 und gegebenenfalls die Beobachtung der Läsionsbildung ermöglicht wird.

Das erfindungsgemässe System bzw. Verfahren ist nicht auf die Untersuchung oder Behandlung des Herzens beschränkt. Es eignet sich auch für andere Organe und/oder andere zweite Signale, die beispielsweise die Bestimmung anderer Parameter, welche mit der Funktion des Organs zusammenhängen, z. B. Druck, Temperatur oder chemische Parameter, ermöglichen.

## Patentansprüche

1. System zur Visualisierung von Aktivitäten eines Organs, insbesondere des Herzens, mit einer bildgebenden Ultraschallvorrichtung (2) zum Bereitstellen von ersten Signalen, mit einer Rekonstruktionseinheit (7), welche aus den ersten Signalen eine bildliche Darstellung des Organs (10) oder von Teilen des Organs erstellt, mit einer Messvorrichtung (3) zum örtlich aufgelösten Erfassen zweiter Signale, insbesondere elektrischer Signale, welche zweiten Signale repräsentativ für die Aktivität des Organs (10) sind, sowie mit einer Zuordnungseinheit (6), welche den zweiten Signalen jeweils als Ortsinformation den örtlichen Bereich des Organs (10) zuordnet, für dessen Aktivität das jeweilige zweite Signal repräsentativ ist, sowie mit einer Visualisierungseinheit (8), welche aus der bildlichen Darstellung, den zweiten Signalen sowie der jeweils zugehörigen Ortsinformation ein Bild des Organs (10) oder von Teilen des Organs (10) erzeugt, wobei das Bild sowohl die bildliche Darstellung als auch die Aktivität des Organs (10) in einander örtlich zugeordneter Weise umfasst.

2. System nach Anspruch 1, wobei die Ultraschallvorrichtung (2) und die Rekonstruktionseinheit (7) derart ausgestaltet sind, dass sie eine dreidimensionale bildliche Darstellung des Organs (10) oder von Teilen des Organs (10) ermöglichen.

3. System nach einem der vorangehenden Ansprüche, wobei die Messvorrichtung (3) zum Erfassen der zweiten Signale mindestens eine Elektrode (41a,41c) zum lokalen Erfassen der elektrischen Aktivität des Organs (10) umfasst.

4. System nach einem der vorangehenden Ansprüche, wobei die Messvorrichtung (3) einen Katheter (4) umfasst, der mindestens eine Kontakt-Elektrode (41a,41c) oder mindestens eine Elektrode (41a,41c) zur kontaktlosen Erfassung der elektrischen Aktivitäten des Organs (10) umfasst.

5. System nach Anspruch 4, wobei der Katheter (4) mindestens einen Ultraschallwandler (44) zum Empfangen und/oder Aussenden von Ultraschallsignalen umfasst.

6. System nach Anspruch 4 oder 5, wobei der Katheter (4) mehrere Elektroden (41a,41c) umfasst, die insbesondere zwei- oder dreidimensional angeordnet sind.

7. System nach einem der Ansprüche 4-6, mit einem Ultraschallwandler (44), der bezüglich des Katheters (4) beweglich angeordnet ist.

8. System nach einem der Ansprüche 5-7, wobei die Ultraschall-Vorrichtung (2) und/oder die Zuordnungseinheit (6) so ausgestaltet ist bzw. sind, dass mit Hilfe von Signalen, die von dem oder den Ultraschallwandler(n) (44) kommen, den zweiten Signalen automatisch die zugehörige Ortsinformation zugeordnet werden kann.

9. System nach einem der Ansprüche 5-8, wobei mindestens ein Ultraschallwandler (44) auch als Elektrode (41a) zum lokalen Erfassen der elektrischen Aktivität dient.

10. System nach einem der Ansprüche 4-9, wobei die örtliche Lage des Katheters (4) in dem Bild enthalten ist.

11. System nach einem der Ansprüche 4-10, wobei mindestens eine Elektrode (41a,41c) auch als Hochfrequenz-Energiequelle für Ablationen ausgestaltet ist.

12. System nach einem der vorangehenden Ansprüche, wobei die zweiten Signale elektrokardiografische (EKG-) Daten umfassen.

13. Verfahren zur Visualisierung von Aktivitäten eines Organs, insbesondere des Herzens, bei welchem mittels Ultraschallmesssignalen eine bildliche Darstellung des Organs (10) oder von Teilen des Organs (10), insbesondere eine dreidimensionale bildliche Darstellung, erzeugt wird, und bei welchem zweite Signale, insbesondere elektrischer Signale, welche repräsentativ für die Aktivität des Organs (10) sind, mit der genannten bildlichen Darstellung zu einem Bild des Organs (10) oder von Teilen des Organs (10) kombiniert werden, wobei das Bild sowohl die bildliche Darstellung als auch die Aktivität des Organs (10) in einander örtlich zugeordneter Weise umfasst.

14. Verfahren nach Anspruch 13, wobei das Bild kontinuierlich oder in diskreten Zeitabständen aktualisiert wird, indem aktuelle zweite Signale mit der bildlichen Darstellung neu kombiniert werden.

15. Verfahren nach einem der Ansprüche 13 oder 14, wobei das Bild kontinuierlich oder in diskreteten Zeitabständen aktualisiert wird, indem die bildliche Darstellung oder Teile davon aktualisiert wird oder werden.
